# EUROPEAN PATENT APPLICATION

(11) **EP 4 714 350 A1**
(43) Date of publication of application: **25.03.2026**
(21) Application number: 24201664.0
(22) Date of filing: 20.09.2024
(51) Int. Cl.: A61B 5/271, A61B 5/276, A61B 5/28, A61B 5/00

(54) **BODY SENSOR UNIT**

(71) Applicant: Koninklijke Philips N.V., 5656 AG Eindhoven (NL)
(72) Inventor: GELISSEN, Jozef Hubertus, Eindhoven (NL)
(74) Representative: Philips Intellectual Property & Standards

(57) **Abstract**

A body sensing unit having a pad part which adheres to the skin and a lead or tail part which electrically connects to the pad part. The lead or tail part is formed by a flat substrate, for example carrying printed electronics, and which has a novel shape which forms a coil or loop, or part thereof, around at least part of the pad part. The novel shape changes the distribution of stress if the tail is displaced, e.g. pulled or tugged, during use, lessening the chance that the pad part becomes detached from the skin.

## Description

### FIELD OF THE INVENTION

The invention relates to the field of body sensing devices, and in particular to the field of body sensing devices employing sensing components which adhere to the skin.

### BACKGROUND OF THE INVENTION

Some types of medical sensing devices use sensing components which have a sensing surface which, in use, faces onto the skin. It may be held onto the skin by a surrounding mounting structure. This may for example include an adhesive surface to adhere to the skin and thereby hold the sensing surface to the skin.

One class of such sensing component is an electrode for sensing a bio-electrical signal, e.g. an ECG electrode or a biopotential sensing electrode. Another class of sensing component is a monitoring patch which includes sensing components of one or more modalities, e.g. ultrasound transducers, skin conductance sensors, PPG sensors or others.

In the case of an electrode, the electrode includes a mounting structure for holding the conductive area of the electrode against the skin. Typically, this is an adhesive structure. For example an adhesive structure may surround a conductive area of the electrode.

One class of electrode is an ECG electrode.

A standard type of ECG electrode comprises an adhesive substrate for adhering to skin, an electrically conductive material for electrically interfacing with skin, e.g. a high viscous salt-solution in the form of a gel, and an electrical connection to an electrical cable which runs to an ECG control unit. The electrical connection between the electrode and the cable may also be a mechanical connection. This mechanical connection may be fixed or may be separable. The connection may be referred to as a mechanical-electrical connection.

There are different types of mechanical-electrical connections.

Two broad categories are: removable connections, and fixed (or integral) connections.

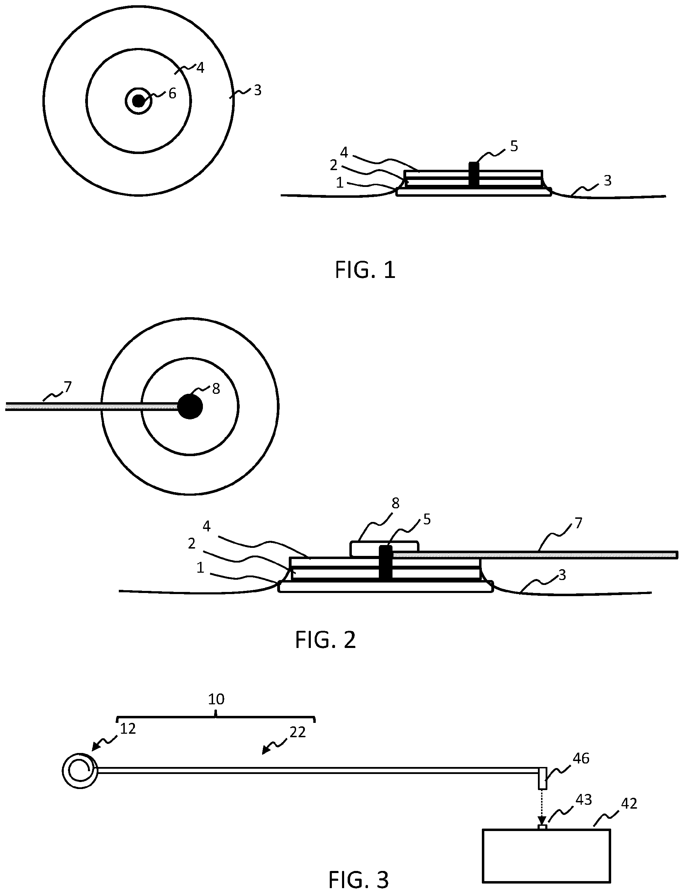

Within the category of removable connection, one type is a snap-fit connector. One example is shown in Fig. 1. This comprises a protrusion 6, e.g. a boss, on the upper exposed surface of the electrode (i.e. the part which couples to the skin). The electrical wire comprises a connector at one end which comprises a depression or groove which is complementary to the protrusion 6 comprised by the electrode upper surface and wherein the protrusion can be pushed into the depression to snap fit the connector to the electrode. The structure of the electrode is shown in cross-section in Fig. 1 (right). The electrode comprises a first plate element 2 which has a bore formed through it, the bore positioned centrally, and wherein a pillar 5 is positioned within the bore extending out-of-plane of the first plate element 2. A substrate layer 3 is placed atop the first plate element 2. The substrate layer comprises an adhesive on its lower surface for adhering to skin. The substrate layer has a larger diameter than the first plate element 1 so that its edges extend over the top of the first plate element to make contact with the skin, for holding the first plate element in place. The substrate layer 3 may for example be formed of a non-woven fabric material. A second plate element 4 placed atop the substrate layer, sandwiching the substrate between the first plate element 2 and the second plate element 4. The pillar 5 protrudes from the top surface of the second plate element 4, and its upper section forms the protrusion 6 mentioned previously. During use, a conductive material 1 is placed between the skin of the user and the first plate element 2. Electrical signals from the body are conducted through the conductive material 1, along the length of the pillar 5 and then into the wire which is connected to the pillar. The conductive material may be a conductive gel for example.

The advantage of this type of connector is structural simplicity and thus low manufacturing cost.

Another type of connector within the category of removable connector is the so called 'tab-electrode'. This type includes a tab at one side of the electrode to which a clamp connector can form a clamp connection.

Within the category of fixed or integral connection, the most common type is the so-called pre-wired electrode. Fig. 2 shows plan and cross-sectional views of an example of a pre-wired electrode. A pre-wired electrode comprises an electrical wire 7 of which one end is fixedly electrically coupled to the electrically conductive material of the electrode. An advantage of this type of connection is greater connection security compared to a removable connection type such as the snap-on connection. Another advantage of this type of connection is that the electrical connection does not require the manual application of pressure in order to establish it. This avoids a need to push hard onto the body of the patient to connect the wire, and also avoids the gradual wear and tear to which a removable connection is subject. This type is often used for long term monitoring and for frail patients (e.g. neonatal patients).

In the standard implementation, the structure of the pre-wired electrode is similar to the snap-on electrode except that the electrical and mechanical coupling between depression and protrusion is fixedly held in place by a permanent cover or cap 8 to keep it in place. A cover layer may be disposed atop the electrode, for example a non-woven material such as a non-woven tape. This gives a more gentle feel for the subject.

With regards to the cable 7, this is typically an insulated electrical cable comprising a conductive core with an electrically insulating sheath, e.g. a plastic sheath. It is typically tubular in construction. One end of the cable electrically connects to the conductive material of the electrode, while a second end of the cable typically connects to a standardized medical connector which can then be coupled to a medical connector interface on e.g. an ECG control unit.

With regards to the electrically conductive material, traditionally this comprises a viscous salt-solution in the form of a gel.

A later innovation is the so-called `dry electrode'. Dry electrodes do not need to use a conductive gel and so are more convenient. In the most common case, the electrically conductive material of a dry electrode may comprise a metal such as stainless steel. Another type is cloth electrodes formed of a conductive fabric woven into a cloth. Another more recent type takes the form of a skin adhesive which is mixed with materials which give the adhesive electrically conductive properties, such as carbon or conductive polymers.

A newer innovation is the use of printed electronics for electrodes and for sensing patches. Conductive ink is printed onto a carrier substrate (e.g. comprising a PET foil) to form electrical connection tracks which can take the place of traditional wires. At the end of each track may be a connection area such as a contact which can electrically interface with the electrically conductive material of the electrode or patch.

The inventors have identified some problems in the current state of the art for skin-mounted body sensors.

The construction of state of the art prewired electrodes is complex and cumbersome to fabricate. Furthermore, the structure of the standard electrodes is such that it is difficult to engage the electrode surface flush against the skin. This results in an uneven contact area between the skin and the electrode, which results in loss of functionality.

Use of printed electronics can improve upon some of these difficulties. It is known in the field of health monitoring patches to use printed electrical circuitry to directly electrically interface with the dry electrode material and to extend outward along a flat tail structure which takes the place of the standard wire. The flat tail carries a printed electrical track for electrically connecting the electrically conductive material of the electrode to a medical connector for connecting to an ECG control unit. This results in an electrode structure with a geometry which is flat and flexible out-of-plane and thus easier to conform to the skin surface.

However, this alternative design creates a new problem. The tail is formed by a flat carrier substrate which carries the electrical connection to the medical connector. The flat geometry of this substrate gives rise to a point of weakness at the point of convergence with the pad area of the electrode which is contact with the skin. When in a flat relaxed state, the tail part extends in a straight line to the periphery of the pad part where it converges into the pad part. However, when pulled or tugged in certain directions, the flat substrate of the tail part acts very much like paper, and preferentially bends at the join line with the pad part. This creates a line at which stress becomes focused which is at the periphery of the pad part. This puts excessive strain on the skin adhesive causing early or even total detachment of the skin adhesive.

### SUMMARY OF THE INVENTION

The invention is defined by the claims.

According to examples in accordance with an aspect of the invention, there is provided a body sensor unit. The body sensor unit comprises a pad part. The pad part carries an electrically conductive material. The pad part is for adhering to skin of a subject during use. The electrically conductive material is arranged for making contact with the skin when the pad part is adhered to the skin. The body sensor unit further comprises a tail part. The tail part extends from the pad part. The tail part carries at least one conductive line. The at least one conductive line extends electrically from the electrically conductive material. The tail part is for being non-adhered to the skin during use. The tail part has a flat geometry. The tail part comprises a flat substrate. The at least one conductive line is carried by the flat substrate. The tail part is configured such that, in a flat relaxed state, an at least section of the tail part has a shape which extends in an at least partial loop around the electrically conductive material carried by the pad part.

An at least partial loop means that it extends in a shape which defines at least part of a loop, for example at least part of a curve, for at least part of an annular shape. It extends around the electrically conductive material.

In other words, the region of the tail part where the tail part meets or converges to the pad part, the tail part, when in a flat relaxed shape, has a shape which is at least part of a loop. The loop can be curved or non-curved.

The claimed design has the surprising effect of relieving stress forces when bending or pulling the cable from a single point close to the skin adhesive towards a more distributed area along the cable. If the tail part is formed so that it extends in a straight path to the pad part, then if the tail part is bent or pulled, the join point with the skin-adhered pad part becomes a focal point for the tensile stress and this results in the adverse effect of the skin-adhered pad part tending to be pulled up away from the skin. The looped shape of the invention has the effect of re-directing or spreading some of the tensile force so that it is not all focused at the join point with the pad and is spread somewhat along the tail part, and also is not focused in a direction which is directly radially toward the middle of the pad part. In particular, in embodiments of the invention, when the tail part is pulled or deflected from its flat relaxed state, the join point or convergence point between the tail part and the pad part will curl around the part of the pad which is adhered to the skin (the skin adhesion element). The point where this curling occurs is different depending on the direction in which the tail part is pulled relative to the pad part.

The at least section of the tail part may extend in only a partial loop, or it may extend in a full loop, or it may extend in more than a full loop, for example it may extend in a coil shape so that it extends around more than one full loop. It may be a spiral shape. It may be an at least partial loop which is curved or non-curved. It may have a shape which is in the form of a non-circular polygon, e.g. forming part of a hexagonal or rectangular loop shape, or any other polygonal shape.

In some embodiments, the tail part meets the pad part at a join location. The said at least section of the tail part is preferably a section of the tail part which extends from the join location with the pad part. In other words, the tail part has a first end and a second end, and wherein a first end of the tail part forms a first end of the said at least section of the tail part which extends in the at least partial loop around the electrically conductive material.

In some embodiments, the at least section of the tail part is shaped and configured such that, in a flat relaxed state, it extends in a curve around at least part of the pad part. In other words, it is curved in shape. In other words, it is round or rounded in shape. In other embodiments, however, it may be a non-round or non-curved loop, such a loop defining part of a square shape or part of a hexagon shape or part of any polygonal shape.

In some embodiments, the at least section of the tail part is shaped and configured such that, in a flat relaxed state, it extends in a spiral around at least part of the pad part. For example, it may extend in a shape which defines at least part of a spiral. For example, it may extend in a shape which defines at least part of a coil. For example, it is coil-shaped.

In some embodiments, in a flat relaxed state, said at least section of the tail part extends around at least half of a periphery of the electrically conductive material.

In some embodiments, in a relaxed flat state, said at least section of the tail part extends around at least ¾ of the periphery of the electrically conductive material, for example at least substantially 100% of the periphery of the electrically conductive material.

In some embodiments, the pad part includes an outer area region which extends around an outer periphery of the electrically conductive material when the pad part is adhered to the skin, and wherein the tail part is configured such that, in a flat relaxed state, said at least section of the tail part rests atop of the outer area region of the pad part.

This avoids the tail part directly touching the skin which avoids skin irritation.

In some embodiments, the outer area region is for adhering to the skin.

In some embodiments, the conductive line is a printed conductive line. In other words, the conductive line is formed by printed electronics. The printed conductive line may be printed onto the flat substrate of the tail part.

In some embodiments, the body sensor unit comprises a skin adhesion element. The skin adhesion element may be annular. A lower surface of the skin adhesion element may carry an adhesive material for adhering to skin. The skin adhesion element may surround the electrically conductive material. A part of the previously mentioned flat substrate may be adhered to an area region of the upper surface of the skin adhesion element. The area region may be a radially inner annular area region of the upper surface of the skin adhesion element. The tail part may be formed by a part of the flat substrate which is not adhered to the radially inner annular area region.

In some embodiments, the flat substrate may be provided with a spiral-shaped cut line extending from the part of the flat substrate which is adhered to the skin adhesion element and extending around the electrically conductive material. The spiral-shaped cut line may form a section of the flat substrate which extends in an at least partial loop shape around the electrically conductive material. The section of the flat substrate which extends in an at least partial loop shape may form the at least section of the tail portion which extends in an at least partial loop.

The spiral shaped cut line for example defines a boundary edge of the at least section of the tail part which extends around the electrically conductive material.

In some embodiments, the flat substrate further carries a conductive contact. In some embodiments, the conductive contact is arranged to make electrical contact with the electrically conductive material when the flat substrate is adhered to the skin adhesion element. One end of the at least one conductive line carried by the flat substrate may be electrically connected to the conductive contact.

In some embodiments, a first end of the tail part is joined to, or converges to, the pad part and a second end of the tail part is connected to an electrical connector for connecting to an ECG control unit.

In some embodiments, the body sensor unit is an electrode for an ECG apparatus.

An aspect of the invention provides a body sensing apparatus. The body sensing apparatus may comprise one or more body sensor units in accordance with any preceding claim. The body sensing apparatus may further comprise a body sensing control unit electrically connected with the one or more body sensor units. The body sensing control unit may comprise a processing device configured to compute biosignal measurement data using electrical signals received from the one or more body sensor units.

In some embodiments, the body sensing apparatus may be an ECG sensing apparatus. The body sensor units may be ECG sensor units, e.g. ECG electrodes. The body sensing control unit may be an ECG control unit. The processing device comprised by the ECG control unit may be configured to compute ECG measurement data using electrical signals received from the one or more body sensor units. In other words, the biosignal data may be ECG measurement data.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a prior art body sensor unit in the form of a snap-fit ECG electrode;
Fig. 2 shows a different prior art body sensor unit in the form of a pre-wired ECG electrode;
Fig. 3 shows an example body sensing apparatus which provides an aspect of the invention and, in the context of which, embodiments of the claimed body sensor unit may be applied;
Fig. 4 shows a part cut-away view of an example body sensor unit in accordance with one or more embodiments, in which upper surfaces of the skin adhesion element and electrically conductive material are visible;
Fig. 5 shows in schematic form a fully assembled view of an upper side of the body sensor unit of Fig. 1;
Fig. 6 shows the tail part of the body sensor unit in accordance with one or more embodiments, in which the at least section of the tail part which extends in loop-like shape is highlighted in grey;
Fig. 7 shows a model of the geometry of a body sensor unit not in accordance with the invention wherein the tail part of the unit joins the pad part in a straight line direction;
Fig. 8 shows a model of the geometry of a body sensor unit in accordance with one or more embodiments of the present invention, wherein the tail portion includes a loop-like section;
Fig. 9 is an exploded view of a layer structure which may be used to form the body sensor unit of Fig. 4 - Fig. 6.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Embodiments of the invention provide a body sensing unit having a pad part which adheres to the skin and a lead or tail part which electrically connects to the pad part. The lead or tail part is formed by a flat substrate, for example carrying printed electronics, and which has a novel shape which forms a coil or loop, or part thereof, around at least part of the pad part. The novel shape changes the distribution of stress if the tail is displaced, e.g. pulled or tugged, during use, lessening the chance that the pad part becomes detached from the skin.

One end of the loop section forms the end of the tail part which joins or converges with the pad part.

The design alleviates stress from the join point with the part of the body sensor unit which adheres to the skin (skin adhesion element). This helps solve the problem of the pad part being pulled off the skin if the tail part is pulled or tugged.

Fig. 3 shows an example body sensing apparatus for use in sensing one or more biosignals. For example, the body sensing apparatus may be an ECG apparatus. The body sensing apparatus comprises at least one body sensor unit 10 which is for attachment to a user's skin for measuring one or more biological signals. The body sensor unit generates electrical signals associated with a sensed bodily signal. The body sensing unit comprises a pad part 12 and a tail part 10. The pad part is for adhering to skin of a subject during use. The pad part may include one or more sensitive elements for measuring the relevant bodily signals. The one or more sensitive elements may be for example one or more electrically conductive elements for measuring electrical signals. The one or more sensitive elements may however instead comprise sensitive elements of other modalities, e.g. ultrasound, PPG, and/or skin conductance. The body sensor unit 20 further includes a tail part 22. The tail part carries an electrically conductive line which is for electrically connecting to the sensitive element of the pad part 12 and carrying the electrical signals generated at the pad part indicative of the sensed bodily signals. The body sensing apparatus further includes an electrical connector 46. The electrical connector may for example be a standardized medical connector. The electrically conductive line is connected at a first end to the sensitive element of the pad part 12 and at the other end to the electrical connector 46. The body sensing apparatus further comprises a body sensing control unit 42. The body sensing control unit for example includes a processing device. The body sensing control unit 42 includes a connection interface 43 for electrically and mechanically connecting to the electrical connector 46. The processing device may be configured to compute one or more biosignals based on electrical signals received at the connection interface 43 from the body sensing unit 10 via the conductive line of the tail part 22. The body sensing apparatus may comprise more than one of the body sensing units 10.

The body sensing apparatus may be an ECG apparatus. The body sensing unit 10 may be an ECG electrode. The body sensing control unit 42 may be an ECG control unit comprising a processing device for computing ECG measurement data based on electrical signals received from the ECG electrode at the connection interface 43.

The ECG apparatus may comprise a plurality of the ECG electrodes 10 and may include a connection interface operable to receive electrical signals independently from all of the ECG electrodes.

Embodiments of the present invention relate to an improvement of the body sensing unit 12. Aspects of the invention include the body sensing unit itself as well as a body sensing apparatus, in accordance with that described above, comprising the body sensing unit.

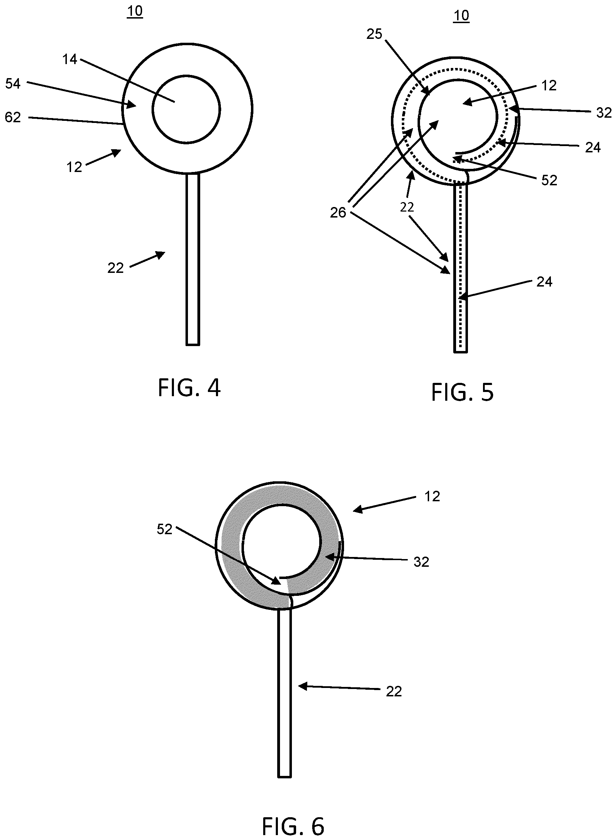

A body sensor unit in accordance with at least one set of embodiments of the present invention is shown in Fig. 4-Fig. 7. The body sensor unit may be an ECG electrode. However, the body sensor unit may be a sensor unit according to any of a range of other modalities.

The body sensor unit 10 comprises a pad part 12. The body sensor unit further comprises a tail part 22. The pad part 12 is for adhering to skin of a subject during use. The tail part 22 extends from the pad part 12.

Fig. 4 shows a part cut-away view of the body sensor unit in which upper surfaces of a skin adhesion element 62 of the pad part and electrically conductive material 14 carried by the pad part are visible. Fig. 5 shows in schematic form a fully assembled view of an upper side of the body sensor unit of Fig. 1 which shows an end section 32 of the tail part which loops around the location of the electrically conductive material 14 before joining or converging to the pad part at a join location 52 radially inset from a periphery of the pad part 12. This section of the tail part 22 rests atop upper surface of the skin adhesion element 62 which is visible in Fig. 4 when the body sensing unit is in an assembled state.

An upper surface is a surface configured to face away from the skin of the subject, when the body sensor unit is worn, i.e., the upper surface is the surface opposite of the surface configured to adhere to the skin of the subject.

The tail part 22 is for being non-adhered to the skin during use. In other words, the tail part is free to be lifted up from the skin and moved. The tail part meets with or converges into the pad part 12 at a join location 52. The join location is radially inset from a periphery of the pad part. Before the join location, the tail part is free to be lifted up away from the body. The join location 52 marks the point at which the tail part meets the part of the body sensor unit 10 which is adhered to the skin during use. This part which is adhered directly to the skin includes at least a region of the pad part 12. For example, the pad part may include a skin adhesion element 62 (mentioned above) which includes an adhesion layer on its bottom surface and which is directly adhered onto the skin during use.

The pad part 12 comprises an electrically conductive material 14 which is arranged for making contact with the skin when the pad part 12 is adhered to the skin. The electrically conductive material is for electrically interfacing with the skin. The electrically conductive material is held onto the skin surface by the skin adhesion element 62. For example, a radially inner annular area of the skin adhesion element 62 overlaps radially with a radially outer annular area portion of the electrically conductive material 14.

The tail part 22 is characterized by a shape which is unique compared with prior art body sensor units. An at least section 32 of the tail part is configured in a shape which forms at least part of a loop or curve or spiral or coil shape. The section 32 of the tail part configured with the at least partial loop shape is shown in Fig. 5 and is highlighted in grey in Fig. 6. This section of the tail part is the section of the tail part which ends at the terminal end of the tail part. In other words, the terminal end section of the tail part, which at one end meets or converges with the pad part 12 is shaped to form a loop or partial loop or curve or spiral shape.

The tail part 22 has a flat geometry and is formed by at least part of a flat substrate 26. The flat substrate is for example bendable out-of-plane. The tail part is resilient in the out-of-plane dimension so that it will or can return to a stable flat relaxed state if released from a bent or deformed position. The tail part is configured such that, in its flat relaxed state, the at least section 32 of the tail part has a shape which extends in an at least partial loop around the electrically conductive material 14 carried by the pad part.

The pad part has an outer area region 54 which extends around the electrically conductive material 14, for example around an outer periphery of the electrically conductive material 14. The outer area region is formed by an upper surface of the skin adhesion element 62. The at least section of the tail part 32 which extends in the loop-like shape may rest atop the outer area region 54 of the pad part 12. The section 32 of the tail part which rests atop the outer area region of the pad part is liftable up away from the pad part, i.e. its surface area is not adhered to the upper surface of the outer area region 54 of the pad part.

In the illustrated example, the tail part is formed by a tail section of a flat substrate layer 26 which is adhered to a radially inner region of an upper surface of the skin adhesion element 62 of the pad part 12 by an annular adhesive layer (or loop) formed on the upper surface of the skin adhesion element 62 between the radially inner edge of the skin adhesion element and the join location 52 between the tail part 22 and the pad part 12. The loop part of the tail section is formed by providing a spiral or loop shaped cut line 25 (or slit) in the part of the flat substrate layer that is not adhered to the skin adhesion element 62.

Fig. 4 shows a partial cut-away view of the body sensor unit 10 in which the flat substrate layer 26 is not applied, while Fig. 5 shows a view of the assembled body sensor unit in which the flat substrate layer 26 is applied. Fig. 4 thus shows the outer area region 54 of the pad part 12 without the terminal loop-like section 32 of the tail part shown. This view shows the upper surface of the outer area region 54 of the pad part 12 which is formed by an upper surface of the skin adhesion element 62. The upper surface of the electrically conductive material 14 is also visible. Fig. 5 shows a view of the upper (top) side of the body sensor unit 10 in a fully assembled state. In this state, the loop-like section 32 of the tail part is shown. It is visible in this view how this section of the tail part terminates at one end at a join location 52 with the pad part, extends in a curl around the periphery of the electrically conductive material 14 along a path which is shaped so that, in a flat relaxed state, the loop-like section 32 of the tail follows the path of the outer area region 54 of the pad part and rests atop and covers the upper surface of the outer area region 54 of the pad part (which in turn is formed by the upper surface of the skin adhesion element 62).

The upper surface of the assembled body sensing unit may further be covered by a cover layer which may provide an insulation effect and may be formed of a material which is softer than the flat substrate atop which it is applied.

The tail part 22 carries at least one conductive line 24 which extends electrically from the electrically conductive material 14. The path of the conductive line is shown schematically in Fig. 5. The conductive line is for example for providing an electrical connection between the electrically conductive material 14 of the pad part 12 and an electrical connector 46, where the electrical connector is for connection to a body sensing control unit 42. A first end of the conductive line 24 is electrically connected to the electrically conductive material 14 of the body sensing unit. A second end of the conductive line 24 may for example be electrically connected to an electrical connector 46.

The at least section 32 of the tail part which extends in the at least partial loop shape may be referred to for brevity as the `loop section' in the forthcoming description. However, more generally, the at least section 32 of the tail part may extend in only a partial loop, or it may extend in a full loop, or it may extend in more than a full loop, for example it may extend in a coil shape so that it extends around more than one full loop. It may be a spiral shape. It may be an at least partial loop which is curved or non-curved. It may have a shape which is in the form of a non-circular polygon, e.g. forming part of a hexagonal or rectangular loop shape, or any other polygonal shape.

The advantage of the loop-like structure 32 at the end of the tail part 22 is that it better distributes stress forces in the event that the tail part is pulled or tugged or bent relative to the pad part. If the tail part extends in a straight path to the pad part, then if the tail part is bent or pulled, the join point with the skin-adhered pad part becomes a focal point for the tensile stress and this results in the adverse effect of the skin-adhered pad part tending to be pulled up away from the skin. The looped shape has the effect of re-directing or spreading some of the tensile force so that it is not all focused at the join point with the pad and is spread somewhat along the tail part, and also is not focused in a direction which is directly radially toward the middle of the pad part 12.

In a flat relaxed state, the loop section extends around the electrically conductive material 14 of the pad part 12. When the tail part is pulled or deflected from its flat relaxed state, the join point 52 or convergence point between the tail part 22 and the pad part 12 will curl around the part of the pad which is adhered to the skin (the skin adhesion element 62). The point where this curling occurs is different depending on the direction in which the tail part is pulled relative to the pad part.

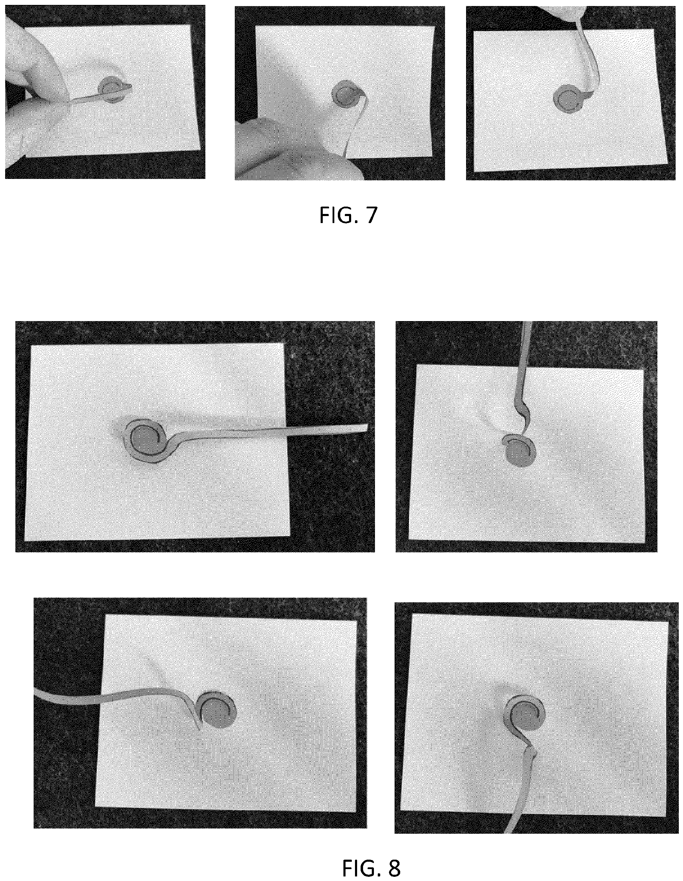

This effect is illustrated in the photographs shown in Fig. 7 and Fig. 8. The photographs in Fig. 7 show a model of the geometry of an example body sensor unit not in accordance with the invention, wherein the tail part joins with the pad part in a straight line direction. There is no loop section to the tail part. The photographs in Fig. 7 illustrate pulling of the tail part in different directions. When pulled in any direction, stress is focused at the join point between the tail part and the pad part, having the effect, if maintained, of pulling the pad part away from the skin.

The photographs in Fig. 8 illustrate the geometry in accordance with the principles of the present invention. The tail part is shaped so that it follows a spiral-like loop path before converging with the pad part. It can be seen that when pulling the tail part, the tail part has a tendency to curl, and the point where this curling occurs is different depending on the direction in which the tail part is pulled relative to the pad part. This results in less focused strain at the point of join or convergence between the tail part and the pad part, and thus less force pulling the pad part away from the skin.

The loop-like section 32 of the tail part 22 may be shaped and configured such that, in a flat relaxed state, it extends in a curve around at least part of the pad part.

The loop-like section 32 of the tail part may be shaped and configured such that, in a flat relaxed state, it extends in a spiral shape around at least part of the pad part.

In a flat relaxed state, the loop-like section 32 of the tail part extends around at least half of the periphery of the electrically conductive material 14.

In flat relaxed state, the loop like section 32 of the tail part may extend around at least ¾ of the periphery of the electrically conductive material, preferably at least substantially 100% of the periphery of the electrically conductive material 14.

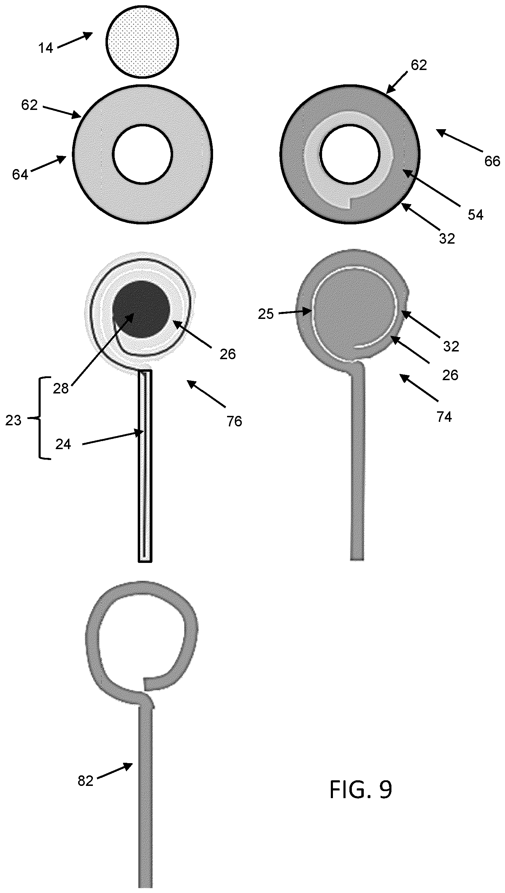

Fig. 9 shows an exploded view of an example layer structure which may be used to form the structure of the body sensor unit 10. The Figures on the right side of Fig. 9 show the top sides of the relevant layer components (i.e. the side facing upward away from the skin when the body sensor unit is applied to the skin in use). The Figures on the left side of Fig. 9 show the bottom sides of the relevant layer components (i.e. the side facing toward the skin when the body sensor unit is attached to the skin). A lower-most layer is the electrically conductive material 14, which may be formed by an electrically conductive material layer. Atop the electrically conductive material 14 is disposed a skin adhesion element 62 formed of a skin adhesion layer. The skin adhesion element 62 is annular in shape, and is disposed atop the electrically conductive material so that a radially inner annular area of the skin adhesion element overlaps radially with a radially outer annular area portion of the electrically conductive material. An upper surface 66 of the skin adhesion element 62 forms the aforementioned outer area region 54 of the pad portion 12. The skin adhesion element 62 surrounds the electrically conductive material 14. A bottom side 64 of the skin adhesion element carries a layer of adhesive material which is for adhering the skin adhesion element to the skin. The adhesive material for example covers the whole of the bottom surface of the skin adhesion element. The skin adhesion element 62 holds the conductive material 14 against the skin during use.

The body sensor unit further comprises a flat substrate layer 26. The lower (skin-facing) side 76 of the flat substrate layer 26 is shown on the left of Fig. 9. The upper side 74 of the flat substrate layer 26 (facing away from the skin) is shown in the right of Fig. 9. The tail part 22 of the body sensor unit 10 is formed by a tail portion of the flat substrate layer 26.

The flat substrate layer 26 carries electrical connection circuitry 23. This may be carried on the bottom, skin-facing side 76 of the flat substrate layer. The electrical connection circuitry 23 includes the electrically conductive line 24 carried by the tail part 22 of the body sensor unit. The electrical connection circuitry further includes a conductive contact 28 which is for making electrical connection with the conductive material. When assembled, the flat substrate layer 26 is disposed atop the top surface 66 of the skin adhesion element 62, with the conductive contact 28 disposed atop, and in electrical contact with, the electrically conductive material 14. One end of the conductive line 24 is electrically connected with the conductive contact part of the electrical connection circuitry 23. The conductive line extends along the surface of the flat substrate layer around the loop section 32 of the tail part and along the non-loop section of the tail part.

The top side 66 of the skin adhesion element has a layer of adhesive covering part of its area, in particular a radially inner-most annular ring area immediately surrounding the electrically conductive material 14. This is for adhering the part of the flat substrate layer 26 carrying the conductive contact 28 onto the pad part so as to hold the conductive contact 28 against the conductive material 14 during use. Thus, the central area of the circular part of the flat substrate area, i.e. the part carrying the conductive contact 28, is adhered to the pad part 12 by a ring of adhesive located at the radially innermost area of the annular skin adhesion element 62, extending around the central opening in the middle of the skin adhesion element. The lighter grey area of the top side 66 of the adhesion layer 62 shown in Fig. 9 corresponds to the area which is covered by the adhesive for adhering to the flat substrate layer 26. This then leaves the radially outer area portion of the flat substrate layer 26 not adhered to the pad part. This part is cut with a spiral-shaped cut line 25, as visible on the upper-side view 74 of the flat substrate layer 26 in Fig. 9. This then forms the loop section 32 of the tail portion 22 of the body sensor unit 10. In other words, the tail part 22 of the of the body sensor unit 10, i.e. the part which can be lifted up freely from the body, is formed by the section of the flat substrate layer 26 which is not adhered onto the skin adhesion element 62.

The body sensor unit may further comprise a cover layer 82 which is for providing an electrical insulation function and covers the exposed top and bottom surfaces of the flat substrate layer 26. This may be formed of a material which is softer than the material of the flat substrate layer onto which it is applied. This provides a gentle feel to the user. For example, it may be formed of a non-woven fabric, or a foam material.

With regards to the flat substrate 26, this may be formed of a PET foil in some examples.

With regards to the skin adhesion element, this may be formed for example by a layer of PET. However, other materials are also possible.

With regards to the electrically conductive material, this may be a dry electrode material. In the most common case, the electrically conductive material of a dry electrode may comprise a metal such as stainless steel. Another type is cloth electrodes formed of a conductive fabric woven into a cloth. Another more recent type takes the form of a skin adhesive which is mixed with materials which give the adhesive electrically conductive properties, such as carbon or conductive polymers.

With regards to the electrical connection circuitry 23, including the conductive line 24, which is carried by the flat substrate layer 26, in some embodiments this may be printed electrical connection circuitry. It may be formed by electrically conductive ink. Thus, the conductive line 24 may be a printed conductive line.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A body sensor unit (10), comprising:
a pad part (12) for adhering to the skin of a subject during use, wherein the pad part carries an electrically conductive material (14), and wherein the electrically conductive material is being arranged for making contact with the skin when the pad part is adhered to the skin;
a tail part (22) for being non-adhered to the skin during use, wherein the tail part extends from the pad part, and wherein the tail part carries at least one conductive line (24) which extends electrically from the electrically conductive material;
wherein the tail part (22) has a flat geometry and comprises a flat substrate (26), wherein the at least one conductive line (24) is carried by the flat substrate;
wherein the tail part (22) is configured such that, in a flat relaxed state, an at least section (32) of the tail part has a shape which extends in an at least partial loop around the electrically conductive material carried by the pad part.

2. The body sensor unit of claim 1, wherein
the tail part meets the pad part at a join location (52); and
said at least section (32) of the tail part is a section of the tail part which extends from the join location (52) with the pad part.

3. The body sensor unit of claim 1 or 2, wherein
the at least section (32) of the tail part is shaped and configured such that, in a flat relaxed state, it extends in a curve around at least part of the pad part.

4. The body sensor unit of any preceding claim, wherein
the at least section (32) of the tail part is shaped and configured such that, in a flat relaxed state, it extends in a spiral around at least part of the pad part.

5. The skin-sensing structure of any preceding claim, wherein
in a flat relaxed state, said at least section of the tail part extends around at least half of a periphery of the electrically conductive material.

6. The body sensor unit of any preceding claim, wherein
in a relaxed flat state, said at least section of the tail part extends around at least ¾ of the periphery of the electrically conductive material, preferably at least substantially 100% of the periphery of the electrically conductive material.

7. The body sensor unit of any preceding claim,
wherein the pad part includes an outer area region (54) which extends around an outer periphery of the electrically conductive material when the pad part is adhered to the skin, and wherein the tail part is configured such that, in a flat relaxed state, said at least section of the tail part rests atop of the outer area region of the pad part.

8. The body sensor unit of claim 7, wherein the outer area region (54) is for adhering to the skin.

9. The body sensor unit of any preceding claim, wherein
the conductive line is a printed conductive line, and
the printed conductive line is printed onto the flat substrate of the tail part.

10. The body sensor unit of any preceding claim,
wherein the body sensor unit comprises a skin adhesion element (62),
wherein the skin adhesion element (62) is annular,
wherein a lower surface of the skin adhesion element carries an adhesive material for adhering to skin,
wherein the skin adhesion element surrounds the electrically conductive material (14);
wherein a part of the flat substrate (26) is adhered to a radially inner annular area region of the upper surface of the skin adhesion element,
wherein the tail part (22) is formed by a part of the flat substrate which is not adhered to the radially inner annular area region.

11. The body sensor unit of claim 10,
wherein the flat substrate (26) is provided with a spiral-shaped cut line (25) extending from the part of the flat substrate which is adhered to the skin adhesion element (62) and extending around the electrically conductive material (14); and
wherein the spiral-shaped cut line (25) forms a section of the flat substrate which extends in an at least partial loop shape around the electrically conductive material, wherein said section of the flat substrate which extends in an at least partial loop shape forms the at least section of the tail part (22) which extends in an at least partial loop.

12. The body sensor unit of claim 10 or 11,
wherein the flat substrate (26) further carries a conductive contact (28);
wherein the conductive contact is arranged to make electrical contact with the electrically conductive material (14) when the flat substrate is adhered to the skin adhesion element (62);
wherein one end of the at least one conductive line (24) carried by the flat substrate is electrically connected to the conductive contact.

13. The body sensor unit of any preceding claim, wherein
a first end of the tail part is joined to the pad part and a second end of the tail part is connected to an electrical connector for connecting to an ECG control unit.

14. The body sensor unit of any preceding claim, wherein the body sensor unit is an electrode for an ECG apparatus.

15. A body sensing apparatus (40), comprising:
one or more body sensor units (10) in accordance with any preceding claim; and
a body sensing control unit (42) electrically connected with the one or more body sensor units,
wherein the body sensing control unit (44) comprises a processing device configured to compute biosignal data using electrical signals received from the one or more body sensor units.
